# EUROPÄISCHE PATENTANMELDUNG

(11) **EP 1 788 503 A2**
(43) Veröffentlichungstag der Anmeldung: **23.05.2007**
(21) Anmeldenummer: 06405463.8
(22) Anmeldetag: 03.11.2006
(51) Int. Cl.: G06F 19/00

(54) **Vorrichtung und Verfahren zur Übermittlung von Daten von mobilen medizinischen Geräten**

(30) Priorität: 16.11.2005 CH 18442005
(71) Anmelder: ARCOMED AG, 8105 Regensdorf (CH)
(72) Erfinder: Vollenweider, Marc, Kirkland, WA 98033 (US)
(74) Vertreter: Liebetanz, Michael

(57) **Zusammenfassung**

Eine Vorrichtung zur Übermittlung von Daten von mobilen medizinischen Geräten (10) an ein Datenverwaltungssystem (1) hat einen Speicher (30) zur Speicherung von pumpenspezifischen Daten und eine Übertragungseinrichtung (12) zur Übermittlung von vorgangsspezifischen Daten an das Datenverwaltungssystem (1). Dabei verfügt die Vorrichtung (11) über eine Steuereinrichtung (13), mit der in dem besagten Speicher (30) auch patienten- und/oder zuordnungsspezifische Daten zum aktuellen Vorgang speicherbar sind, und mit der zu vorbestimmten oder bestimmbaren Zeitpunkten diese patienten- und/oder zuordnungsspezifische Daten an das Datenverwaltungssystem (1) übertragbar sind, um sie dort dem zugehörigen patientenrelevanten Datensatz (2) zuzuordnen. Dies gestattet die lückenlose Übernahme von patienten- und/oder zuordnungsspezifische Daten, insbesondere vom Beginn einer Notversorgung oder bei Unterbrüchen der Leitung (20) zu dem Datenverwaltungssystem (1).

## Beschreibung

### Technisches Gebiet der Erfindung

Die Erfindung betrifft eine Vorrichtung zur Übermittlung von Daten von mobilen medizinischen Geräten an ein Datenverwaltungssystem, mit einem Speicher zur Speicherung von pumpenspezifischen Daten und mit einer Übertragungseinrichtung zur Übermittlung von vorgangsspezifischen Daten an das Datenverwaltungssystem, und ein Verfahren hierfür.

### Stand der Technik

Aus der DE 10 2004 011 168 A1 ist ein medizinisches Versorgungssystem bekannt, welches insbesondere dafür ausgelegt ist, Daten von mehreren medizinischen Geräten und insbesondere von mehreren Infusionspumpen zu verarbeiten, dabei auch fähig ist, Daten von Beatmungsgeräten und von solchen Geräten zu verarbeiten, die lebenswichtige Daten, sogenannte Vital Signs, aufzeichnen.

Die DE 10 2004 011 168 offenbart dabei eine zentrale Prozessorvorrichtung, auf der eine Anwendung läuft, die von den angeschlossenen Pumpen fluidinfusionsbezogene Daten über eine beispielsweise TCP/IP-Übertragung erhält. Diese Daten werden von der Anwendung entweder kontinuierlich aber auch nicht kontinuierlich, nämlich periodisch oder nicht periodisch, abgefragt, und in der zentralen Prozessorvorrichtung erfasst, verarbeitet und mit dem betreffenden Patienten korreliert in der besagten zentralen Datenbank abgespeichert.

Ein solches Verfahren und eine solche Vorrichtung bezieht sich auf die Verfolgbarkeit der Daten und den sicheren Datenaustausch in klinischen Umgebungen, der immer wichtiger wird. In der Medizin kommen immer mehr und mehr technische Geräte in den Einsatz und es wird immer schwieriger den kompletten Überblick des aktuellen Zustandes für den einzelnen Patienten und über alles zu behalten.

Die US 6,790,198 B1 offenbart eine Vorrichtung, die über drahtlose Kommunikation pumpenbezogene Daten an ein solches Krankenhaus-Informations-Management-System liefert und dort mit anderen Daten integriert.

Im Bereich der Infusionen und Verabreichung von Medikamenten werden mehr und mehr Infusionsgeräte benutzt, die programmiert und dann kontrolliert dem Patienten Medikamente zuführen. Diese Infusionsgeräte sind mit Kommunikationsschnittstellen ausgerüstet, so dass die Programmierung und Ereignisse wie Starten, Stoppen, Ratenwechsel, Alarme usw. während der Infusion registriert werden können und diese an ein solches oben genanntes System weitergeleitet werden.

Der gesamte Datenaustausch wird allgemein häufig unter dem Begriff PDMS für Patientendaten-Management-System (aus dem Englischen für Patient Data Management System) zusammengefasst. Ein anderer Begriff in diesem Zusammenhang ist HIMS für Krankenhaus-Informations-Management-System (aus dem Englischen für Hospital Information Management System).

PDM-Systeme sind im heutigen Gebrauch administrative Programme, die den Ablauf im Spital vereinfachen. Beginnend mit der Registrierung eines Patienten, Verordnen von Medikamenten bis zur Rechnungsstellung werden patientenspezifische Daten zugeordnet. Einige Systeme haben auch Lagerverwaltungsmodule und Lohnmodule, um den internen Spital- oder Klinikablauf zu verwalten.

### Zusammenfassung der Erfindung

Diese bekannten Vorrichtungen sind aber nicht fähig, neben dem aktuellen Zustand auch die vergangenen Ereignisse aufzulisten, die zum aktuellen Zustand geführt haben. Es ist bei Ihnen auch nicht möglich, zu einem späteren Zeitpunkt die Daten aus der Vergangenheit aufzubereiten und den genauen Ablauf wieder zu rekonstruieren.

Ausgehend von diesem Stand der Technik ist es damit eine Aufgabe der vorliegenden Erfindung eine Vorrichtung und ein Verfahren anzugeben, welches diese Funktionalität bietet.

Ferner ist der Stand der Technik nicht in der Lage, die Anforderung zu erfüllen, dass auch ein Protokoll von verwendeten Geräten im Bezug mit dem Patienten in diese Datenbank aufgenommen werden kann. Röntgenbilder, CTI- und MR-Scans sollten direkt in die Patienten-spezifische Datenbank eingebunden werden, wie auch Laborresultate, so dass der Arzt einen direkten und schnellen Zugriff auf alle Daten hat.

Diese Aufgaben und weitere sich beim Stand der Technik ergebende Probleme werden mit einer Vorrichtung und einem Verfahren nach den beigefügten Ansprüchen gelöst.

Eine gelöste Problematik liegt darin, dass Infusionsgeräte sehr mobil sind und trotzdem die Daten sicher und eindeutig Patienten zugeordnet werden müssen. Bei herkömmlichen Systemen müssen die Infusionsgeräte zur Einbindung immer am Originalstandort bleiben. Eine Lösung zur Erhöhung der Mobilität ist aus der US 6,790,198 B1 entnehmbar, bei der durch drahtlose Kommunikation der Daten auch bei bewegten Infusionsgeräten diese Übermittlung allgemein gewährleistet werden soll.

Diese Übertragung ist aber bei Unterbrüchen in der Übertragung durch Abschirmungseffekte, bei Überlastung des drahtlosen Funknetzes oder anderen Gründen nicht mehr sicher.

Mit der erfindungsgemässen Lösung sind die Daten rückverfolgbar, so dass nach der Einbindung der Geräte in ein System die ganze Aufzeichnung hochflexibel ist.

Die hier beschriebene Vorrichtung und Verfahren erlaubt die Einbindung von Infusionsgeräten oder anderen mobilen Geräten in einer Art, dass die Daten sicher dem Patienten zugeordnet werden können ohne an Flexibilität der Geräte zu verlieren.

Damit ist ein sicheres und automatisches Tracking und Update von Pumpendaten unabhängig vom aktuellen Standort der Pumpe möglich. Die Pumpen oder andere medizinische Geräte sind jederzeit und unabhängig vom Standort lokalisierbar können und die Daten können gegebenenfalls in praktisch Echtzeit zurück zum Patienten zurückverfolgt werden, auch wenn die Pumpen oder Geräte nicht mehr am Bett (Standort des Patienten) betrieben werden.

Ein Vorteil der erfindungsgemässen Lösung liegt darin, dass sehr viele Geräte, insbesondere Pumpen (beispielsweise über 500 Geräte) in einem Netzwerk gleichzeitig laufen. Oft werden dann auch gleichzeitig an mehreren Terminals Daten von viele Pumpen (ein Patient kann beispielsweise bis zu 10 Pumpen aufweisen) abgefragt werden. Das kann zu im Umfang enormen Datentransfers führen, so dass das System sehr langsam wird.

Vorteilhafterweise werden gemäss Ausführungsformen der Erfindung nur neue 'Events' übertragen, so dass nicht bei jeder Abfrage die ganze History von jeder Pumpe über das Netzwerk übertragen werden muss.

Ein weiterer wesentlicher Vorteil liegt darin, dass Pumpen oder andere medizinische Geräte jederzeit aus dem System entfernt werden können (zum Beispiel beim Transport oder einer Verlegung), wobei während der Abwesenheit weiter Events generiert werden, die beim Einfügen wieder automatisch in das Verwaltungssystem nachgeführt werden. Dieses 'inkrementelle' Updaten der Daten passiert nicht nur zwischen den Pumpen und der direkten Interfaceeinrichtung zur Pumpe, sondern auch zwischen der direkten Interfaceeinrichtung und dem zentralen Rechnersystem.

Insbesondere zeigt sich die Vorteilhaftigkeit des Systems bei der Notfallversorgung. Es ist in der Praxis oft der Fall, dass ein Gerät aktiviert und gestartet wird, bevor der Patient registriert ist. Dies gilt sowohl beim Einsatz in der Notaufnahme als auch draussen, an einem Unfallort oder bei einem lebensbedrohliche Beschwerden aufweisenden Patienten zu Hause. Mit der Inbetriebnahme der Infusionspumpe oder eines anderen medizinischen Geräts, welches beispielsweise Herzdaten oder andere vitale Daten erfasst, startet die Datenaufzeichnung, lange bevor der Patient in einem PDMS oder HIMS aufgenommen worden ist. Ein solches System erkennt bei Eintreffen des oder der Geräte im Krankenhaus, entweder drahtlos (IR, Funk oder RFID) oder drahtgestützt, dass diese Pumpen oder Geräte noch nicht zugeordnet sind. Die in dem oder den Geräten gespeicherten Daten können dann zeitverschoben zugeordnet und ausgelesen werden.

Ferner kann einem Kommunikationsstandort beispielsweise automatisch ein Bett und/oder Patient zugeordnet werden. Dies kann die Zuordnung der Daten vereinfachen, wobei alle Geräte, die an einem solchen Standort am laufen sind und noch keine Zuordnung haben, diesem Bett und/oder Patient zugeordnet werden und dem Anwender, zumeist also einem Arzt oder einer Krankenschwester, die Möglichkeit gegeben wird, diese Zuordnung zu bestätigen oder abzuweisen.

Zusammenfassend sind die Vorteile der vorliegenden Vorrichtung und des Verfahrens, dass Datenverluste sicher vermieden werden können, dass eine eindeutige Zuordnung Gerät⇔Patient gewährleistet ist, dass ein rasches Auffrischen der Daten im zentralen Speicher des Datenverwaltungssystems des Krankenhauses trotz der verlangten Mobilität möglich ist, dass Kommunikationsunterbrüche kein Problem darstellen, und dass ein solches System leicht skalierbar ist, also viele mobile medizinische Geräten im Verbund gestattet.

Die vorgeschlagene Lösung, wo die Pumpe oder das mobile Gerät einen Teil der Zuordnung zum Patienten und die Information, was noch zu übertragen ist, intern speichert, hat auf einen Schlag persistente und jahrelange Probleme des Standes der Technik mit grossen Gerätepools gelöst, denn diese Vorgehensweise gestattet die lückenlose Übernahme von patienten- und/oder zuordnungsspezifische Daten, insbesondere vom Beginn einer Notversorgung oder bei Unterbrüchen der Leitung zu dem Datenverwaltungssystem.

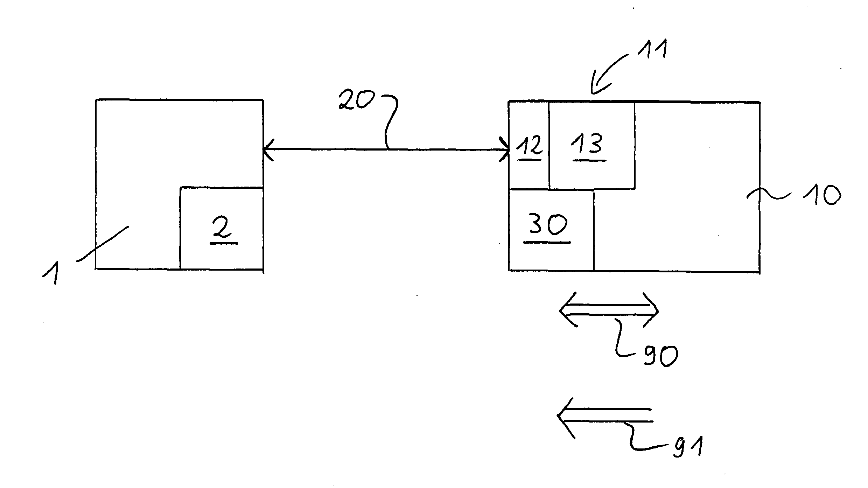

### Kurze Beschreibung der Zeichnung

Die einzige Figur zeigt in sehr schematischer Weise eine Vorrichtung gemäss einem Ausführungsbeispiel der Erfindung.

### Ausführliche Beschreibung eines Ausführungsbeispiels der Erfindung

Im Krankenhaus besteht ein Datenverwaltungssystem 1, welches über Speicher 2 verfügt, in denen patienten- und/oder zuordnungsspezifische Daten abgelegt sind. Solche Daten können Abrechnungsdaten, medizinische Befunde, Verordnungen, Rezepte, Angaben zu durchgeführte Untersuchungen, aber eben auch aufgezeichnete Daten der Krankheitsgeschichte umfassen, wie EKGs oder Infusionshistorien.

Mit dem Bezugszeichen 10 ist ein solches mobiles medizinisches Gerät bezeichnet. Dabei kann es sich beispielsweise um eine Infusionspumpe handeln oder ein ein EKG aufzeichnendes Gerät. Es können Thermometer sein oder sonstige mobile medizinische Apparate zur Überwachung und/oder Versorgung eines Patienten. Diese können zeitweise ein- oder ausgeschaltet sein.

Das mobile medizinische Gerät umfasst eine Vorrichtung 11 zur Übermittlung von Daten von diesem Gerät 10 an das Datenverwaltungssystem 1. Dabei ist mit dem Doppelpfeil 20 eine Leitung bezeichnet, mit der von einer Übertragungseinrichtung 12 vorgangsspezifische Daten an das Datenverwaltungssystem 1 übermittelt werden. Diese Übertragungseinrichtung 12 wird von einer separaten oder mit ihr integrierten Steuereinrichtung 13 kontrolliert, die die entsprechenden Daten aus einem Speicher 30 abgefragt und übermittelt werden.

Hier ist es dem Fachmann im Hinblick auf den Stand der Technik sofort klar, dass es sich bei der Leitung 20 um ein beliebiges Vehikel zur Datenübertragung handeln kann. Dies kann in nicht beschränkender Weise drahtlose Funkübertragungsmodi wie sogenanntes Wireless LAN oder Bluetooth umfassen, aber natürlich auch Infrarotübertragung oder drahtgebundene Übertragung über eine Vielzahl von Protokollen. Es kann sich um drahtgestütztes TCP/IP handeln oder auch um über die Spannungsversorgung geführte Kommunikation.

Wesentlich ist, dass die Vorrichtung 11 mit Hilfe Ihrer Steuereinrichtung 13 auch patienten- und/oder zuordnungsspezifische Daten zum aktuellen Vorgang kontinuierlich oder diskret in Echtzeit oder zeitversetzt in dem Speicher 30 speichert.

Damit werden ortsunabhängig mit dem Patienten und dem Gerät zusammenhängende Ereignisse oder Events aufgezeichnet. Das kann eine Veränderung des körperlichen Zustandes (beispielsweise die Herzfrequenz oder die Temperatur) sein, das kann bei versorgenden Geräten und Einrichtungen die Änderung der Medikation (beispielsweise Erhöhung der Durchflussrate oder Stoppen der Medikamentenverabreichung) sein oder sich ergebende Störfälle technischer oder medizinischer Art. Alle diese und weitere Ärzte- oder Pflegepersonal interessierende Werte werden unter dem Begriff zuordnungsspezifische Daten zum aktuellen Vorgang verstanden und zusammengefasst.

Diese sich ergebenden Daten werden von der Vorrichtung 11 im Speicher 30 niedergelegt. Zu von der Steuereinrichtung 13 vorbestimmten Zeitpunkten werden diese patienten- und/oder zuordnungsspezifische Daten von dem Gerät 10 an das Datenverwaltungssystem 1 übertragen, um sie dort dem zugehörigen patientenrelevanten Datensatz im Speicher 2 zuzuordnen.

Neben der Übertragung zu von der Steuereinrichtung 13 vorbestimmten Zeitpunkten (beispielsweise alle 10 Minuten, wenn eine Verbindung 20 besteht oder aufgebaut werden kann) kann die Übertragung auch am Gerät selbst initialisiert werden, entweder manuell oder beispielsweise durch den Aufbau der Verbindung 20 (beispielsweise Eintritt in ein Funk- oder Bluetooth-Netz oder Einstöpseln des Gerätes an eine leitungsgebundene Schnittstelle).

Damit ergibt sich ein System zur sicheren und automatischen Verfolgen und Aktualisieren von Daten von mobilen Geräten 10, wobei im mobilen Gerät 10 nicht nur die pumpenspezifischen Daten gespeichert werden, sondern auch zuordnungsspezifische Daten zum aktuellen Vorgang, was erlaubt, die Daten unabhängig vom Standort des Gerätes jederzeit wieder dem im Datenmanagementsystem 1 zugehörigen Datensatz im Speicher 2 zuzuordnen.

Es ist klar, dass es damit möglich wird, der notwendigen Mobilität der Geräte 10 Rechnung zu tragen und einen dadurch auftretenden temporären Kommunikationsverlust mit dem Zentralen System 1 aufzufangen. Dies ist durch den die Bewegung anzeigenden Doppelpfeil 90 angezeigt. Das Zwischenspeichern der vorgangsbezogenen Werte im Speicher 30 vermeidet Datenverlust und ermöglicht, wie unten gesehen werden wird, ein effizientes Update und Auffrischen der Datensätze im zentralen Speicher 2. Denn diese Vorrichtung bietet die eindeutige Zuordnung der Patientendaten, auch wenn die Kommunikation (Leitung 20) unterbrochen wird.

Es wird festgehalten, dass in dem mobilen Gerät 10 unabhängig davon, ob es mit dem Datenverwaltungssystem 1 verbunden ist oder nicht, alle relevanten Daten (Vorgangsdaten) gespeichert werden. Diese Daten werden sofort oder zu einem oder mehreren späteren Zeitpunkten in das Datenverwaltungssystem 1 partiell oder komplett übertragen. Bei einer vorteilhaften Ausgestaltung der Erfindung werden jeweils nur die Daten übermittelt, die noch nicht übertragen worden sind. Dies ist insbesondere vorteilhaft, wenn mit einer Vielzahl von beispielsweise 200, 500 oder mehr Geräten parallel gearbeitet werden muss, da vermieden werden kann, dass dauernd ganze Historien-Logfiles übermittelt werden müssen.

Dafür ist in der Steuereinrichtung 13 in dem Speicher 30 des mobilen Gerätes 10 die Information speicherbar, welche Daten bereits sicher an das Datenverwaltungssystem 1 übertragen worden sind. Dann kann nur noch der inkrementelle Unterschied an Daten übertragen werden, wobei nach Bestätigung der erfolgten Übertragung die Information der nun bereits übertragenen Daten gespeichert wird. Darüber hinaus kann dann Speicherplatz von bereits gesendeten Daten für die Aufzeichnung neuer Daten freigegeben werden.

Es werden somit weiterhin neue Datenzugänge während des Übertragens oder Kommunikationsunterbrüche automatisch im mobilen Gerät 10 oder/und im Datenverwaltungssystem 1 registriert, wobei bei der nächsten Übertragung nur die Daten übertragen werden müssen, die noch nicht übertragen wurden oder nicht korrekt übertragen werden konnten.

Alternativ oder zusätzlich kann in dem Speicher 30 ein Schwellwert gespeichert werden, so dass nach Erreichen oder Überschreiten eines solchen Schwellwertes einer Menge an neuen vorgangsbezogenen Daten übertragen wird, sofern eine Leitung 20 besteht oder aufbaubar ist. Prinzipiell kann solch ein Leitungscheck auch nach vorbestimmten Zeitabläufen (zum Beispiel alle 10 Minuten, alle Stunde, jeweils um eine geräteabhängige Zeit nach einer bestimmten Uhrzeit etc.) vorgenommen werden, so dass diese Daten jeweils dann partiell oder komplett an das Datenverwaltungssystem 1 übertragen werden.

Hierfür ist vorzugsweise die Steuereinrichtung 13 mit einer Leitungstestfunktion ausgestattet, um die Abwesenheit des Bestehens einer Leitung 20 zu dem Datenverwaltungssystem 1 feststellen zu können, um nach Herstellen und Feststellen des Bestehens der besagten Verbindung eine Übertragung der gespeicherten patienten- und/oder zuordnungsspezifischen Daten zum aktuellen Vorgang zu initialisieren.

Da man nun nur noch über die Kommunikationsschnittstelle verifizieren muss, welche Daten schon übertragen wurden und zu wem diese Daten gehören, kann mit einem erfindungsgemässen System der sogenannte Overhead so minimiert werden, dass die Übertragung auch bei 200 Pumpen jeweils noch in Echtzeit erfolgt. Diese Lösung, wobei die Pumpe oder das mobile Gerät 10 diese Informationen (wie Zuordnung und auch, was bereits ausgelesen wurde und was nicht) mit sich trägt, löst viele Probleme des Standes der Technik, insbesondere bei grösseren Systemen mit einer Vielzahl von mobilen Geräten.

Vorteilhafterweise verfügt die Steuereinrichtung 13 über eine Eingabemöglichkeit, um dem Gerät 10 einen Standort oder einen Kommunikationsport für eine Datenzuordnung zuzuweisen. Diese Eingabemöglichkeit kann eine raumabhängige Zuordnung sein, beispielsweise über eine IP-Adresse einer Netzwerksteckdose, und somit auch zu einem Patienten, der in dem betreffenden Bettstandplatz eingebucht ist. Es kann auch eine bettabhängige Zuordnung sein, wenn das Bett an sich einem Patienten zugeordnet ist und das Gerät 10 an einer dem Bett zugeordneten Schnittstelle oder Steuereinrichtung eincheckt.

Hierbei ist es vorteilhaft, dass der Steuereinrichtung 13 vom Datenverwaltungssystem 1 ein Aufforderungssteuersignal übermittelbar ist, die Datenzuordnung eines noch nicht zugeordneten mobilen Gerätes 10 einzugeben oder standortbezogen zu bestätigen, wenn also beispielsweise der Patient noch nicht bekannt war, oder eine neue Pumpe, ein (neues) Gerät 10, an einem neuen Ort eingesetzt wird.

Solche Fälle können insbesondere bei Notfällen eintreten, wenn eine Ambulanz einen Verunfallten erstversorgt. Es werden dann Infusionspumpen 10 und andere hochmobile Geräte angeschlossen, die natürlich in Bezug auf diesen Patienten noch überhaupt nicht an ein Datenverwaltungssystem 1 angeschlossen gewesen sind. Dies ist in der Figur durch den Einzelpfeil 91 dargestellt, wo ein fremdes oder neues Gerät in den Bereich eines Datenverwaltungssystems 1 kommt und es erstmalig neue Daten zu dieser Patientenversorgung über die Leitung 20 übermittelt. Durch die erfindungsgemässe Ausgestaltung und Vorgehensweise sammeln die Geräte 10 wertvolle Daten, sowohl in medizinischer als auch in abrechnungstechnischer Hinsicht, die dann anschliessend an das Datenverwaltungssystem 1 übertragen werden können.

Diese Vorgehensweise ist dann insbesondere auch bei der Verlegung von Patienten von einem Krankenhaus in ein anderes vorteilhaft, da die beim Transport anfallenden Daten direkt beim neuen Versorgungsplatz eingebucht werden können.

Für eine einfachere Verwaltung des Gerätepools kann vom Datenverwaltungssystem 1 an die Steuereinrichtung 13 ein Steuersignal übermittelt werden, um die Datenzuordnung eines noch nicht zugeordneten mobilen Gerätes 10 standortbezogen automatisch zuzuordnen, oder um die Zuordnung eines mobilen Gerätes 10 während oder nach Abschluss eines Vorgangs automatisch oder mit Anwenderbestätigung zu löschen.

### Bezugszeichenliste

- 1: Datenverwaltungssystem
- 2: Speicher
- 10: mobiles medizinisches Gerät
- 11: Vorrichtung
- 12: Übertragungseinrichtung
- 13: Steuereinrichtung
- 20: Leitung
- 30: Speicher
- 90: Bewegung des Geräts
- 91: Neuaufnahme eines Geräts

## Patentansprüche

1. Vorrichtung zur Übermittlung von Daten von mobilen medizinischen Geräten (10) an ein Datenverwaltungssystem (1), mit einem Speicher (30) zur Speicherung von pumpenspezifischen Daten und mit einer Übertragungseinrichtung (12) zur Übermittlung von vorgangsspezifischen Daten an das Datenverwaltungssystem (1), **dadurch gekennzeichnet, dass** die Vorrichtung (11) über eine Steuereinrichtung (13) verfügt, mit der in dem besagten Speicher (30) auch patienten- und/oder zuordnungsspezifische Daten zum aktuellen Vorgang speicherbar sind, und mit der zu vorbestimmten oder bestimmbaren Zeitpunkten diese patienten- und/oder zuordnungsspezifische Daten an das Datenverwaltungssystem (1) übertragbar sind, um sie dort dem zugehörigen patientenrelevanten Datensatz (2) zuzuordnen.

2. Vorrichtung gemäss Anspruch 1, **dadurch gekennzeichnet, dass** mit der Steuereinrichtung (13) die Information in dem Speicher (30) des mobilen Gerätes (10) speicherbar ist, welche Daten bereits sicher an das Datenverwaltungssystem (1) übertragen worden sind, und/oder dass nach Erreichen eines Schwellwertes einer Menge an neuen vorgangsbezogenen Daten und/oder nach vorbestimmten Zeitabläufen diese Daten partiell oder komplett an das Datenverwaltungssystem (1) übertragen werden.

3. Vorrichtung gemäss Anspruch 1 oder 2, **dadurch gekennzeichnet, dass** mit der Steuereinrichtung (13) die Abwesenheit des Bestehens einer Leitung (20) zu dem Datenverwaltungssystem (1) feststellbar ist und nach Herstellen und Feststellen des Bestehens der besagten Verbindung eine Übertragung der gespeicherten patienten- und/oder zuordnungsspezifischen Daten zum aktuellen Vorgang initialisierbar ist.

4. Vorrichtung nach einem der Ansprüche 1 bis 3, **dadurch gekennzeichnet, dass** die Vorrichtung eine Anzeige aufweist, die von der Steuereinrichtung (13) ansteuerbar ist, um die gespeicherten Daten auf dem Gerät (10) anzuzeigen.

5. Vorrichtung nach einem der Ansprüche 1 bis 4, **dadurch gekennzeichnet, dass** mit der Steuereinrichtung (13) dem Gerät (10) ein Standort oder Kommunikationsport für eine Datenzuordnung zuweisbar ist.

6. Vorrichtung nach einem der Ansprüche 1 bis 5, **dadurch gekennzeichnet, dass** der Steuereinrichtung (13) vom Datenverwaltungssystem (1) ein Aufforderungssteuersignal übermittelbar ist, die Datenzuordnung eines noch nicht zugeordneten mobilen Gerätes (10) einzugeben oder standortbezogen zu bestätigen.

7. Vorrichtung nach einem der Ansprüche 1 bis 5, **dadurch gekennzeichnet, dass** der Steuereinrichtung (13) vom Datenverwaltungssystem (1) ein Steuersignal übermittelbar ist, um die Datenzuordnung eines noch nicht zugeordneten mobilen Gerätes (10) standortbezogen automatisch zuzuordnen.

8. Vorrichtung nach einem der Ansprüche 1 bis 7, **dadurch gekennzeichnet, dass** der Steuereinrichtung (13) vom Datenverwaltungssystem (1) ein Steuersignal übermittelbar ist, um die Zuordnung eines mobilen Gerätes (10) während oder nach Abschluss eines Vorgangs automatisch oder mit Anwenderbestätigung zu löschen.

9. Verfahren zur Übermittlung von Daten von mobilen medizinischen Geräten (10) an ein Datenverwaltungssystem (1), mit den Verfahrensschritten:
- des Speicherns von sowohl pumpenspezifischen Daten als auch patienten- und/oder zuordnungsspezifischen Daten zum aktuellen Vorgang in einem Speicher (30) des mobilen medizinischen Gerätes (10),
- des Übertragens der besagten gespeicherten Daten an das Datenverwaltungssystem (1) zu vorbestimmten oder bestimmbaren Zeitpunkten, um sie dort dem zugehörigen patientenrelevanten Datensatz (2) zuzuordnen.

10. Verfahren nach Anspruch 9, **dadurch gekennzeichnet, dass** dem Schritt des Übertragens ein weiterer Speicherschritt folgt, in dem die bereits erfolgreich übertragenen Daten gegenüber noch nicht oder nicht erfolgreich übertragenen Daten markiert werden.
